# EUROPEAN PATENT SPECIFICATION

(11) **EP 1 854 354 B1**
(45) Date of publication and mention of the grant of the patent: **24.10.2012**
(21) Application number: 06714716.5
(22) Date of filing: 27.02.2006
(51) Int. Cl.: A01N 43/16, A01N 25/00, A01N 37/40, A01P 3/00, A61K 8/60, A61K 8/73

(54) **AGENT FOR ENHANCING ANTISEPTIC POWER**
MITTEL ZUR ERHÖHUNG DER ANTISEPTISCHEN WIRKSAMKEIT
AGENT DESTINE A AMELIORER UN POUVOIR ANTISEPTIQUE

(30) Priority: 03.03.2005 JP 2005058634
(43) Date of publication of application: 14.11.2007
(73) Proprietor: Kao Corporation, Chuo-ku Tokyo 103-8210 (JP)
(72) Inventor: UENO, Katsuya, Minato, Wakayama-shi, Wakayama, 6408580 (JP); MIZUSHIMA, Hiromoto, Minato, Wakayama-shi, Wakayama, 6408580 (JP); KUBOTA, Hiromi, kabane, Ichikai-machi, Haga-gun, Tochigi (JP); ENDO, Katsumi, Bunka, Sumida-ku, Tokyo, 1318501 (JP)
(74) Representative: HOFFMANN EITLE
(86) International application number: PCT/JP2006/303579
(87) International publication number: WO 2006/100874

(56) References cited:
- EP-A1- 0 551 674
- WO-A1-86/04899
- GB-A- 2 362 320
- JP-A- 05 043 403
- JP-A- 2004 175 846
- US-A- 4 920 100
- US-H- H 224

## Description

### FIELD OF THE INVENTION

The present invention relates to the use of preservative efficacy-enhancing agents for enhancing the efficacy of an antiseptic agent and a method for enhancing a preservative efficacy of antiseptic agents.

### BACKGROUND OF THE INVENTION

In the products such as cosmetics, drugs, quasi-drugs and foods, in order to ensure antiseptic and mildew-proof properties of these products upon production and storage thereof, antiseptic agents such as p-oxybenzoic acid esters (also referred to merely as "parabenes"), benzoic acid and salts thereof, salicylic acid and salts thereof, 2-phenoxyethanol and polyhydric alcohols have been conventionally blended in the products.

Among these antiseptic agents, in particular, the parabenes have been generally used in the applications such as cosmetics because of a high effectiveness thereof. However, some users having a sensitive skin have frequently complained about irritating feel to skin against these antiseptic agents. Also, in the application fields such as cosmetics, there tends to be an increasing demand for products which are gentle and mild for skin and exhibit a still higher safety. For this reason, it has been attempted to reduce an amount of the antiseptic agents such as parabenes and 2-phenoxyethanol which are blended in the cosmetics, etc.

For example, there have been proposed the external preparations containing 1,2-pentanediol and 2-phenoxyethanol (JP 10-53510A); sterile power-enhancing agents having a cation charge which contain sugar alcohols and/or sugars as effective ingredients (JP 10-330793A); antiseptic bactericides using combination of parabenes and 1,2-alkane diols such as 1,2-pentanediol and 1,2-hexanediol (JP 11-310506A); antiseptic bactericides using 1,2-alkane diols or combination of the 1,2-alkane diols, parabenes, 2-phenoxyethanol, etc. (JP 11-322591A); antibacterial mildew-proof assistants using pentose or a sugar alcohol thereof as an effective ingredient (JP2002-302404A); and antiseptic compositions containing an antiseptic agent and 1,2-decanediol (JP 2004-352688A)

In addition, it is known that when 1,3-propanediol is used in combination with an antiseptic agent such as parabenes, an antibacterial glycerol derivative (such as, for example, monofatty acid glycerol esters and monofatty acid polyglycerol esters), a lower alcohol, etc., the amount of the antiseptic agent or antibacterial compound blended can be reduced (JP 2005-15401A).

Thus, there have been conventionally reported various methods for reducing the amount of antiseptic agents blended. However, even when the antiseptic agents are used in combination with other substances, the resultant compositions have still failed to exhibit a satisfactory effect of enhancing a preservative efficacy. Therefore, at present, there is no method for effectively reducing a total amount of the antiseptic agents used.

Also, it is known that when allowing parabenes to coexist with a surfactant, the parabenes are generally incorporated into micelle formed by the surfactant in water, thereby lowering an effective concentration thereof (inactivation phenomenon). The inactivation phenomenon of the parabenes is also recognized in an emulsion system such as an oil-in-water (O/W) emulsion. However, there has been conventionally present no method of effectively controlling or suppressing the inactivation phenomenon.

EP 0551674 relates to oral hygiene antiplaque compositions which include as an active ingredient a glylceroglycolipid having an amine or ether linkage and containing a beta -D-galactose moiety. The glyceroglycolipids are said to provide enhanced inhibition of bacterial adhesive interactions and may provide a surfactancy benefit and/or antibacterial benefit.

GB 2 362 320 describes liquid bactericidal cleaning composition comprising a C₈₋₂₂-alkyl polyglycoside surfactant, a surface active quaternary ammonium salt, and, as the as antibacterial agents, a non halogenated phenyl-phenol compound and Dichlosan. The polyglycoside is said to improve the antibacterial effect.

US H 224 H describes compositions comprising glycosides of formula R(OG)ₓ wherein R is a moiety having 1 to 30 carbon atoms, G is a glycoside, and x indicates the polymerization degree and can range from 1 to 18. The glycoside may be a galactoside. The compositions are for agricultural substates and may contain fungicides or bactericides as active ingredients.

WO 86/04899 concerns ethoxylated glycoside surfactants comprising molecules of the formula R (OG) (EO)_{y} and R (OG)ₓ(EO)_{y}, wherein R is a residue with about 10 to 20 carbon atoms and OG may represent a galactoside.

US 4,920,100 relates to alkyl glycosides such as glucosides in admixture with bactericidally active alcohols or carboxylic acids. The glycosides are said to potentiate the microbicidal effect thereof.

### SUMMARY OF THE INVENTION

Thus, the present invention relates to the following aspects [1] and [2]:
[1] Use of an amphiphilic galactose derivative (A) as an effective ingredient for enhancing the preservative efficiency of an antiseptic agent (B) in a preservative efficacy-enhancing composition,
   wherein the amphiphilic galactose derivative (A) is an alkyl galactoside represented by the following formula (1):

   R-O-(G)ₙ (1)

   wherein R is a linear or branched alkyl group having 6 to 36 carbon atoms which may be substituted; G is a galactose residue; and n is an integer of 1 to 20; and
   wherein the antiseptic agent (B) is at least one compound selected from the group consisting of C₁₋₄ alkyl esters of p-oxybenzoic acid, benzoic acid, alkali metal salts of benzoic acid and phenoxyethanol.
[2] A method for enhancing a preservative efficacy of an antiseptic composition, comprising the step of allowing 0.01 to 30% by mass of an amphiphilic galactose derivative (A) to coexist with an antiseptic agent (B) in the composition
   wherein the amphiphilic galactose derivative (A) is an alkyl galactoside represented by the following formula (1):

   R-O-(G)ₙ (1)
wherein R is a linear or branched alkyl group having 6 to 36 carbon atoms which may be substituted; G is a galactose residue; and n is an integer of 1 to 20; and
wherein the antiseptic agent (B) is at least one compound selected from the group consisting of C₁₋₄ alkyl esters of p-oxybenzoic acid, benzoic acid, alkali metal salts of benzoic acid and phenoxyethanol.

### DETAILED DESCRIPTION OF THE INVENTION

The present invention relates to a preservative efficacy-enhancing agent capable of allowing the antiseptic agent to exhibit a high preservative efficacy when used at a low concentration in antiseptic systems requiring an antiseptic agent; a preservative efficacy-enhancing composition containing such a preservative efficacy-enhancing agent; and a method for enhancing a preservative efficacy of the antiseptic agent.

The present inventors have found that when an amphiphilic galactose derivative having no preservative efficacy by itself is used in combination with an antiseptic agent such as parabenes, the antiseptic agent can be remarkably enhanced in preservative efficacy and the amount of the antiseptic agent blended can be considerably reduced even in systems in which a surfactant inactivating the antiseptic agent is present, resulting in a less irritativeness and a sufficient antiseptic effect.

Thus, the present invention relates to:
[1] Use of an amphiphilic galactose derivative (A) as an effective ingredient for enhancing the preservative efficiency of an antiseptic agent (B) in a preservative efficacy-enhancing composition,
   wherein the amphiphilic galactose derivative (A) is an alkyl galactoside represented by the following formula (1):

   R-O- (G)ₙ (1)

   wherein R is a linear or branched alkyl group having 6 to 36 carbon atoms which may be substituted; G is a galactose residue; and n is an integer of 1 to 20; and
   wherein the antiseptic agent (B) is at least one compound selected from the group consisting of C₁₋₄ alkyl esters of p-oxybenzoic acid, benzoic acid, alkali metal salts of benzoic acid and phenoxyethanol.
[2] A method for enhancing a preservative efficacy of an antiseptic composition, comprising the step of allowing 0.01 - to 30% by mass of an amphiphilic galactose derivative (A) to coexist with an antiseptic agent (B) in the composition
   wherein the amphiphilic galactose derivative (A) is an alkyl galactoside represented by the following formula (1):

   R-O- (G)ₙ (1)

   wherein R is a linear or branched alkyl group having 6 to 36 carbon atoms which may be substituted; G is a galactose residue; and n is an integer of 1 to 20; and
   wherein the antiseptic agent (B) is at least one compound selected from the group consisting of C₁₋₄ alkyl esters of p-oxybenzoic acid, benzoic acid, alkali metal salts of benzoic acid and phenoxyethanol.

### (Preservative Efficacy-Enhancing Agent)

The preservative efficacy-enhancing agent of the present invention contains an amphiphilic galactose derivative (A) as an effective ingredient. The "preservative efficacy-enhancing agent" used herein means those compounds having an effect of enhancing a preservative efficacy of an antiseptic agent when allowing the compounds to coexist with the antiseptic agent even though the compounds have no or very slight preservative efficacy by themselves.

Also, the "preservative efficacy" used herein means a defensive power against all contaminant organisms such as bacteria, mildew and yeast, and also involves a concept of mildew-proofing.

The amphiphilic galactose derivative (A) used in the present invention is represented by the following formula (1):

R-O-(G)n (1)

wherein R is a linear or branched alkyl group having 6 to 36 carbon atoms which may be substituted; G is a galactose residue; and n is an integer of 1 to 20.

The alkyl galactosides represented by the above formula (1) are compounds in which one or more galactose residues are bonded to an alkyl group having 6 to 26 carbon atoms through an α- or β-glycoside linkage.

The alkyl group R in the above formula (1) may be either linear or branched, and has preferably 8 to 22 carbon atoms and more preferably 10 to 20 carbon atoms. Specific examples of the alkyl group R include n-hexyl, n-heptyl, n-octyl, n-decyl, n-dodecyl, n-tetradecyl, n-hexadecyl, n-octadecyl, isostearyl, 2-ethylhexyl, 2-hexyldecyl, 2-octyldodecyl and 2-decyltetradecyl.

Among these alkyl groups, in view of exhibiting a good effect as an antiseptic assistant, preferred are n-decyl, n-dodecyl, n-tetradecyl, n-hexadecyl, 2-hexyldecyl and 2-octyldodecyl.

The galactose residue may include any of a pyranose type, a furanose type and a mixture thereof.

The suffix n is preferably an integer of from 1 to 10 and more preferably from 1 to 6. The (G)n may be a monosaccharide, a disaccharide, a trisaccharide, a tetrasaccharide or the other oligosaccharide. The bonding type of these compounds may be either a maltose type or a trehalose type.

Suitable examples of the alkyl galactosides represented by the above formula (1) include α,β-n-decyl galactoside, α,β-n-dodecyl galactoside, α,β-n-tetradecyl galactoside, α,β-n-hexadecyl galactoside, α,β-2-hexyldecyl galactoside and α,β-2-octyldodecyl galactoside. These alkyl galactosides may be used alone or in combination of any two or more thereof.

The alkyl galactosides represented by the above formula (1) may be produced by the method of subjecting D-galactose sequentially to acetylation, halogenation, alcohol condensation and deacetylation [refer to Ryohei HORI and Yoshihiko IKEGAMI, "Studies on Carbohydrate Derivatives. V. Syntheses of Alkyl Galactosides and Alkyl Glycosides", Pharmaceutical Journal, Vol. 79, No. 1, pp. 80-83 (1959)], the method described below in Production Examples, etc.

### (Preservative Efficacy-Enhancing Composition and Method for Enhancing Preservative Efficacy)

The preservative efficacy-enhancing composition of the present invention contains 0.01 to 30% by mass of an amphiphilic galactose derivative (A) and 0.01 to 1.0% by mass of an antiseptic agent (B). Also, the method for enhancing a preservative efficacy according to the present invention is characterized by allowing 0.01 to 30% by mass of the above amphiphilic galactose derivative (A) to coexist with the antiseptic agent (B).

The "preservative efficacy-enhancing composition" used herein means such a composition containing the antiseptic agent whose preservative efficacy is enhanced by adding the amphiphilic galactose derivative (A) thereto

The antiseptic agent (B) used in the present invention means the following agents added to cosmetics, drugs, quasi-drugs, detergents, foods, etc., for the purpose of suppressing growth of microbes and preventing microbial deterioration of these products, and generally includes those ordinarily used as antiseptics as well as substances having antibacterial or bactericidal properties.

The antiseptic agent (B) usable in the present invention is selected from p-oxybenzoic C₁₋₄ -alkyl esters (parabenes), benzoic acid, salicylic acid, sorbic acid, dehydroacetic acid, p-toluenesulfonic acid and salts thereof, and phenoxyethanol.

The p-oxybenzoic esters (parabenes) are lower alkyl esters thereof containing an alkyl group having 1 to 4 carbon atoms. Examples of the p-oxybenzoic esters (parabenes) include methyl p-oxybenzoate (methyl parabene), ethyl p-oxybenzoate (ethyl parabene) and butyl p-oxybenzoate (butyl parabene). The salts of benzoic acid, salicylic acid, sorbic acid, dehydroacetic acid and p-toluenesulfonic acid are alkali metal salts and preferably sodium salts.

These antiseptic agents (B) may be used alone or in combination of any two or more thereof.

Among these antiseptic agents (B), in view of enhancing a preservative efficacy thereof when used in combination with the amphiphilic galactose derivative (A), preferred are p-oxybenzoic esters, benzoic acid, alkali metal salts of benzoic acid and phenoxyethanol, and in view of a reduced amount of the antiseptic agent used and a low irritativeness to skin, more preferred are one or more compounds selected from the group consisting of p-oxybenzoic esters such as methyl p-oxybenzoate and ethyl p-oxybenzoate, and 2-phenoxyethanol.

Examples of the substances having antibacterial or bactericidal properties include glycerol derivatives, alkane diols and lower alcohols.

Specific examples of the glycerol derivatives include monofatty acid glycerol esters, monofatty acid polyglycerol esters and monoalkyl glyceryl ethers.

The monofatty acid glycerol esters are preferably monoglycerol esters of saturated fatty acids having 6 to 14 carbon atoms and preferably 8 to 12 carbon atoms. Examples of the monofatty acid glycerol esters include monocaprylic acid glycerol ester, monocapric acid glycerol ester and monolauric acid glycerol ester.

The monofatty acid polyglycerol esters are preferably esters of a saturated fatty acid having 6 to 14 carbon atoms and preferably 8 to 12 carbon atoms and polyglycerol (having a polymerization degree of 2 to 12 and preferably 3 to 10). Examples of the monofatty acid polyglycerol esters include monolauric acid pentaglycerol ester and monolauric acid decaglycerol ester.

Examples of the monoalkyl glyceryl ethers include monooctyl glyceryl ether and mono-2-ethylhexyl glyceryl ether.

Among these compounds, especially preferred are monocaprylic acid glycerol ester, monocapric acid glycerol ester, monolauric acid decaglycerol ester, monolauric acid pentaglycerol ester and monooctyl glyceryl ether.

The alkane diols are preferably those having 2 to 12 carbon atoms. Examples of the alkane diols include ethanediol, 1,2-propanediol, 1,3-propanediol, 1,2-butanediol, 1,3-butanediol, 1,4-butanediol, 2,3-butanediol, 1,6-hexanediol and 1,12-dodecanediol. Among these alkane diols, especially preferred are those having 2 to 6 carbon atoms.

The lower alcohols are preferably those alcohols having 1 to 4 carbon atoms. Examples of the lower alcohols include ethanol, n-propanol, isopropanol and various kinds of butanols.

The content of the amphiphilic galactose derivative (A) used in the preservative efficacy-enhancing composition and the method for enhancing a preservative efficacy according to the present invention, is from 0.01 to 30% by mass, preferably from 0.05 to 5% by mass and more preferably from 0.05 to 2% by mass.

The amount of the antiseptic agent (B) blended in the preservative efficacy-enhancing composition may be appropriately determined according to features of commercial products and a necessary preservative efficacy therefor, and is usually from 0.01 to 1.0% by mass and preferably from 0.05 to 0.8% by mass. In the present invention, by using the antiseptic agent (B) in combination with the amphiphilic galactose derivative (A), an adequate preservative efficacy can be maintained, and the amount of the antiseptic agent used can be considerably reduced. Therefore, the preservative efficacy-enhancing composition of the present invention can be used in relief and with a high safety even when applied to persons having a sensitive skin.

In general, an effectiveness of antiseptics tends to be considerably deteriorated in the presence of a nonionic surfactant. However, in the preservative efficacy-enhancing composition and the method for enhancing a preservative efficacy according to the present invention, by blending an adequate amount of the nonionic surfactant together with the amphiphilic galactose derivative (A) and the antiseptic agent (B) in the composition, a remarkable effect of suppressing deterioration of the preservative efficacy can be attained, thereby maintaining a high preservative efficacy.

The nonionic surfactant usable in the present invention may include ordinary nonionic surfactants. Examples of the nonionic surfactant include alkyleneoxide adducts such as polyoxyethylene alkyl ethers, polyoxyethylene hydrogenated castor oil, fatty acid polypropylene glycol esters and fatty acid polyoxyethylene glycol esters, and polyol fatty acid esters such as fatty acid sucrose esters, fatty acid glycerol esters, fatty acid sorbitan esters and fatty acid polyglycerol esters.

The amount of the nonionic surfactant (C) blended may be appropriately determined depending upon the preservative efficacy required for the antiseptic agent (B). In the composition containing from 0.01 to 30% by mass of the amphiphilic galactose derivative (A) and from 0.01 to 1.0% by mass of the antiseptic agent (B), the amount of the nonionic surfactant (C) blended therein is usually from 0.01 to 50% by mass and preferably from 0.1 to 30% by mass.

The preservative efficacy-enhancing agent and the preservative efficacy-enhancing composition of the present invention can be effectively applied to various extensive compositions and products requiring a preservative efficacy. Examples of the compositions and products include foundation cosmetics, make-up cosmetics, other cosmetics such as hair cosmetics, drugs such as external preparations for skin, quasi-drugs, detergents, foods, etc.

Further, the preservative efficacy-enhancing agent and the preservative efficacy-enhancing composition of the present invention may also contain adequate amounts of other components and additives ordinarily added thereto, such as, for example, liquid or solid oils, higher fatty acids, alcohols, surfactants, thickening agents, pH modifiers, humectants, colorants and perfumes depending upon the applications thereof, unless the addition thereof adversely affects the objects of the present invention.

The configuration of the preservative efficacy-enhancing agent and the preservative efficacy-enhancing composition of the present invention upon use is not particularly limited. The preservative efficacy-enhancing agent and the preservative efficacy-enhancing composition of the present invention may be used in various configurations such as an aqueous solution system, a solubilized system, an emulsified system, a gel system, a paste system, an ointment system, an air-sol system, a water-oil two-layer system and a water-oil-powder three-layer system.

### EXAMPLES

### PRODUCTION EXAMPLE 1: Production of α,β-n-dodecyl galactoside

D-galactose and n-dodecyl alcohol were reacted with each other while heating and dehydrating under reduced pressure in the presence of a catalytic amount of p-toluenesulfonic acid monohydrate. The resultant mixture was purified by a silica gel column, thereby obtaining n-dodecyl galactoside having a galactose condensation degree of 1 to 3. As a result of analyzing the obtained product by gel permeation chromatography, gas chromatography and ¹H-NMR, it was confirmed that the thus obtained α,β-n-dodecyl galactoside had an average sugar condensation degree of 1.48, and the n-dodecyl monogalactoside component thereof had such a composition in which a molar ratio of pyranoside to furanoside (pyranoside/furanoside) was 83/17, and a molar ratio of α-pyranoside to β-pyranoside (α/β) in the component was 75/25.

### PRODUCTION EXAMPLE 2: Production of α,β-2-hexyldecyl galactoside

The same procedure as in Production Example 1 was repeated except for using hexyldecyl alcohol as the raw material, thereby obtaining α,β-2-hexyldecyl galactoside.

### EXAMPLE 1 AND COMPARATIVE EXAMPLES 1 AND 2: Antiseptic composition

Using the α,β-n-dodecyl galactoside obtained in Production Example 1, the antiseptic compositions (pH: 6.5) having the formulation (% by mass) shown in the following Table 1 were respectively prepared, and then subjected to the following antibacterial test. The results are shown in Table 1.

### Antibacterial Test

The following bacteria were suspended in a physiological saline such that the number of bacteria or spores was 10⁸ CFU/mL. to prepare a bacteria-containing solution. Then, 0.005 mL of the thus obtained bacteria-containing solution was inoculated into 5 mL of the composition to be tested such that the bacteria concentration therein was 10⁸ CFU/mL. The thus inoculated composition was held at 30°C for a predetermined period of time to measure the change in number of bacteria with time by the calculation using a colony counting method. The antibacterial property of the composition was evaluated according to the following four ratings.

### Kinds of Bacteria Used

Staphylococcus aureus IFO13276
Escherichia coli IFO3972
Pseudomonas aeruginosa IFO13275

### Evaluation Criteria for Antibacterial Properties

**⊚:** Sufficiently high antibacterial property (number of bacteria was decreased by 10⁴ or more)
○ : High antibacterial property (number of bacteria was decreased by 10³ to 10²)
Δ: Low antibacterial property (number of bacteria was decreased by 10² to 10¹)
× : No antibacterial property (number of bacteria was substantially unchanged or rather increased)

**TABLE 1**

| | Example 1 | Comparative Example 1 | Comparative Example 2 |
|---|---|---|---|
| Methyl p-oxybenzoate | 0.075 | 0.075 | - |
| α,β-n-dodecyl galactoside | 0.1 | - | 0.1 |
| Purified water | Balance | Balance | Balance |
| Evaluation of antibacterial property after 9 days | ⊚ | Δ | × |

From the results shown in Table 1, it was confirmed that α,β-n-dodecyl galactoside having an amphiphilic property exhibited no antibacterial property by itself, but was capable of enhancing a preservative efficacy of the antiseptic agent (methyl p-oxybenzoate) when used in combination therewith.

### EXAMPLE 2 AND COMPARATIVE EXAMPLES 3 TO 6: Antiseptic composition

Using the α,β-2-hexyldecyl galactoside obtained in Production Example 2, the antiseptic compositions (pH: 6.5) having the formulation (% by mass) shown in the following Table 2 were respectively prepared, and then subjected to the antibacterial test in the same manner as in Example 1. The results are shown in Table 2.

**TABLE 2**

| | Example | Comparative Examples | | | |
|---|---|---|---|---|---|
| | 2 | 3 | 4 | 5 | 6 |
| Methyl p-oxybenzoate | 0.15 | 0.15 | 0.15 | - | 0.15 |
| α,β-2-hexyldecyl galactoside | 0.1 | - | - | 0.1 | - |
| Polyoxyethylene (60) hydrogenated castor oil | 1.0 | - | 1.0 | 0.1 | 1.0 |
| α,β-2-hexyldecyl glycoside | - | - | - | - | 0.1 |
| Purified water | Balance | Balance | Balance | Balance | Balance |
| Evaluation of antibacterial property after 7 days | ⊚ | ○ | Δ | × | Δ |

From the results shown in Table 2, it was confirmed that α,β-2-hexyldecyl galactoside having an amphiphilic property exhibited no antibacterial property by itself, but was capable of specifically enhancing a preservative efficacy of the antiseptic agent (methyl p-oxybenzoate) inactivated by the nonionic surfactant (polyoxyethylene (60) hydrogenated castor oil) when used in combination with the antiseptic agent.

### EXAMPLE 3 AND COMPARATIVE EXAMPLES 7 TO 9: Antiseptic composition

Using the α,β-2-hexyldecyl galactoside obtained in Production Example 2, the antiseptic compositions (pH: 6.5) having the formulation (% by mass) shown in the following Table 3 were respectively prepared, and then subjected to the following antiseptic test. The results are shown in Table 3.

### Antiseptic Test

The following microbe were suspended in a physiological saline such that the number of microbe or spores was 10⁸ CFU/mL. to prepare a microbe-containing solution. Then, 0.005 mL of the thus obtained microbe-containing solution was inoculated into 50 g of the composition to be tested such that the microbe concentration therein was 10⁸ CFU/mL. The inoculated composition was held at 30°C for 28 days to measure the change in number of microbe with time by the calculation using a colony counting method. The antiseptic property of the antiseptic composition was evaluated according to the following four ratings.

### Kinds of Microbe Used

Staphylococcus aureus IFO13276
Escherichia coli IFO3972
Pseudomonas aeruginosa IFO13275
Aspergillus niger IFO6341
Penicillium citrinum IFO6352
Cladosporium cladosporoides IFO6348

### Evaluation Criteria for Antiseptic Properties

⊚: High antiseptic property
○: Some antiseptic property
Δ: Substantially no antiseptic property
× : No antiseptic property

**TABLE 3**

| | Example 3 | Comparative Examples | | |
|---|---|---|---|---|
| | | 7 | 8 | 9 |
| Methyl p-oxybenzoate | 0.2 | 0.2 | 0.2 | 0.2 |
| α,β-2-hexyldecyl galactoside | 0.1 | - | - | - |
| Polyoxyethylene (60) hydrogenated castor oil | 0.5 | 0.5 | 1.0 | 0.1 |
| Galactose | - | 0.1 | - | - |
| Lactose | - | - | 0.1 | - |
| Extract of thujopsis (hiba arborvitae) | 3.0 | 3.0 | 3.0 | 3.0 |
| Purified water | Balance | Balance | Balance | Balance |
| Evaluation of antiseptic property | ⊚ | Δ | Δ | Δ |

From the results shown in Table 3, it was confirmed that only α,β-2-hexyldecyl galactoside having an amphiphilic property was capable of enhancing a preservative efficacy of the antiseptic agent (methyl p-oxybenzoate).

### EXAMPLES 4 AND 5 AND COMPARATIVE EXAMPLES 10 AND 11: Antiseptic composition

Using the α,β-2-hexyldecyl galactoside obtained in Production Example 2, the antiseptic compositions (pH: 6.5) having the formulation (% by mass) shown in the following Table 4 were respectively prepared, and then subjected to the antibacterial test in the same manner as in Example 1. The results are shown in Table 4.

**TABLE 4**

| | Example 4 | Comparative Example 10 | Example 5 | Comparative Example 11 |
|---|---|---|---|---|
| Methyl p-oxybenzoate | 0.15 | 0.15 | 0.15 | 0.15 |
| α,β-2-hexyldecyl galactoside | 0.1 | - | 0.1 | - |
| Polyoxyethylene octyldodecyl ether | 1.0 | 1.0 | - | - |
| EMALEX RWIS-160^{*1} | - | - | 1.0 | 1.0 |
| Purified water | Balance | Balance | Balance | Balance |
| Evaluation of antibacterial property after 10 days | ○ | × | ⊚ | × |

| | | | | |
|---|---|---|---|---|
| Note *1: Nonionic surfactant available from Nihon Emulsion Co., Ltd.; polyoxyethylene monoisostearate hydrogenated castor oil (40 E.O.) | | | | |

From the results shown in Table 4, it was confirmed that α,β-2-hexyldecyl galactoside having an amphiphilic property was capable of enhancing a preservative efficacy of the antiseptic agent (methyl p-oxybenzoate) inactivated by the nonionic surfactant (EMALEX RWIS-160).

### EXAMPLE 6 AND COMPARATIVE EXAMPLES 12 TO 14

Using the α,β-2-hexyldecyl galactoside obtained in Production Example 2, the milky lotions (emulsions) having the formulation (% by mass) shown in the following Table 5 were respectively prepared by the following method. The resultant milky lotions were held at a pH of 6.5 and 30°C for 14 days, and then subjected to an antiseptic test in the same manner as in Example 3. The results are shown in Table 5.

### (Production of Milky lotions)

A solution prepared by dissolving the component (10) in the component (9) at room temperature was added to an aqueous solution prepared by uniformly dissolving the components (11) to (15), (17) and (18) under heating. The obtained solution was slowly added to a solution prepared by dissolving the components (1) to (8) and (16) under heating. The resultant mixed solution was stirred using a homomixer, and then cooled to prepare a milky lotion.

**TABLE 5**

| | | Example 6 | Comparative Examples | | |
|---|---|---|---|---|---|
| | | | 12 | 13 | 14 |
| Formulation | | | | | |
| 1 | Sodium polyoxyethylene lauryl ether phosphate | 0.6 | 0.6 | 0.6 | 0.6 |
| 2 | Sorbitan monostearate | 0.3 | 0.3 | 0.3 | 0.3 |
| 3 | Sodium N-stearoyl-N-methyl taurine | 0.8 | 0.8 | 0.8 | 0.8 |
| 4 | Polyglyceryl diisostearate | 1.0 | 1.0 | 1.0 | 1.0 |
| 5 | Olive oil | 2.0 | 2.0 | 2.0 | 2.0 |
| 6 | Squarane | 0.5 | 0.5 | 0.5 | 0.5 |
| 7 | Cetyl alcohol | 1.0 | 1.0 | 1.0 | 1.0 |
| 8 | Stearyl alcohol | 0.5 | 0.5 | 0.5 | 0.5 |
| 9 | Methyl polysiloxane (6 CS) | 5.0 | 5.0 | 5.0 | 5.0 |
| 10 | Glycerol | 15 | 15 | 15 | 15 |
| 11 | Carboxyvinyl polymer *3 | 0.2 | 0.2 | 0.2 | 0.2 |
| 12 | Succinic acid | 0.01 | 0.01 | 0.01 | 0.01 |
| 13 | Potassium hydroxide | 0.07 | 0.07 | 0.07 | 0.07 |
| 14 | Methyl p-oxybenzoate | 0.3 | 0.3 | 0.3 | 0.3 |
| 15 | 1,3-Butylene glycol | 3.0 | 3.0 | 3.0 | 3.0 |
| 16 | α,β-2-hexyldecyl galactoside | 0.1 | - | - | - |
| 17 | Galactose | - | - | 0. 1 | - |
| 18 | Lactose | - | - | - | 0.1 |
| 19 | Purified water | Balance | Balance | Balance | Balance |
| Evaluation of antiseptic property | | ⊚ | Δ | Δ | Δ |

| | | | | | |
|---|---|---|---|---|---|
| Note *3: "CARBOPOLE 981" (tradename) available from Noveon Inc. | | | | | |

From the results shown in Table 5, it was confirmed that the α,β-2-hexyldecyl galactoside having an amphiphilic property was capable of enhancing a preservative efficacy of the milky lotion, and the obtained milky lotion had a less irritativeness to skin.

### EXAMPLE 7 AND COMPARATIVE EXAMPLE 15

Using the α,β-2-hexyldecyl galactoside obtained in Production Example 2, the emulsions having the formulation (% by mass) shown in the following Table 6 were respectively prepared by the following method. The resultant emulsions were held at a pH of 6.5 and 30°C for 14 days, and then subjected to an antiseptic test in the same manner as in Example 3. The results are shown in Table 6.

### (Production of Cream)

A solution prepared by dissolving the component (12) in the component (11) at room temperature was added to an aqueous solution prepared by uniformly dissolving the components (13) to (18) and (20) under heating. The obtained solution was slowly added to a solution prepared by dissolving the components (1) to (10) and (19) under heating. The resultant mixed solution was stirred using a homomixer, and then cooled to prepare a emulsion.

**TABLE 6**

| | | Example 7 | Comparative Example 15 |
|---|---|---|---|
| Formulation | | | |
| 1 | N-(hexadecyloxyhydroxypropyl)-N-hydroxyethyl hexadecanamide | 3.0 | 3.0 |
| 2 | Polyoxyethylene sorbitan monostearate (20 E.O.) | 0.4 | 0.4 |
| 3 | Sorbitan monostearate | 0.6 | 0.6 |
| 4 | Sodium polyoxyethylene lauryl ether phosphate | 0.6 | 0.6 |
| 5 | Polyglycerol diisostearate | 2.0 | 2.0 |
| 6 | Cholesteryl isostearate | 10.0 | 10.0 |
| 7 | Cetanol | 1.5 | 1.5 |
| 8 | Stearyl alcohol | 1.0 | 1.0 |
| 9 | Squarane | 3.0 | 3.0 |
| 10 | Dipropylene glycol | 3.0 | 3.0 |
| 11 | Methyl polysiloxane (10 CS) | 5.0 | 5.0 |
| 12 | Glycerol | 15.0 | 15.0 |
| 13 | Carboxyvinyl polymer *3 | 0.5 | 0.5 |
| 14 | Potassium hydroxide | 0.25 | 0.25 |
| 15 | Methyl p-oxybenzoate | 0.3 | 0.3 |
| 16 | Extract of brown aglae (phaeophyceace) | - | 0.2 |
| 17 | Extract of burnet | - | 0.3 |
| 18 | Extract of thujopsis (hiba arborvitae) | - | 1.0 |
| 19 | α,β-2-hexyldecyl galactoside | 0.1 | - |
| 20 | Perfume | trace | trace |
| 21 | Purified water | Balance | Balance |
| Evaluation of antiseptic property | | ○ | × |

| | | | |
|---|---|---|---|
| Note *3: "CARBOPOLE 981" (tradename) available from Noveon Inc. | | | |

From the results shown in Table 6, it was confirmed that the emulsion obtained in Example 7 which contained α,β-2-hexyldecyl galactoside having an amphiphilic property exhibited a strong preservative efficacy, and the resultant emulsion had a less irritativeness to skin.

### INDUSTRIAL APPLICABILITY

In accordance with the present invention, when the amphiphilic galactose derivative is allowed to coexist with an antiseptic agent such as parabenes, an preservative efficacy of the antiseptic agent can be remarkably enhanced, and a total amount of the antiseptic agent used can be effectively reduced. Also, even when the antiseptic agent is used at a low concentration and a surfactant coexists therewith, it is possible to allow the antiseptic agent to exhibit a sufficient preservative efficacy and, as a result, obtain products having a low irritativeness to skin.

## Claims

1. Use of an amphiphilic galactose derivative (A) as an effective ingredient for enhancing the preservative efficiency of an antiseptic agent (B) in a preservative efficacy-enhancing composition,
wherein the amphiphilic galactose derivative (A) is an alkyl galactoside represented by the following formula (1):
R-O- (G)ₙ (1)
wherein R is a linear or branched alkyl group having 6 to 36 carbon atoms which may be substituted; G is a galactose residue; and n is an integer of 1 to 20; and
wherein the antiseptic agent (B) is at least one compound selected from the group consisting of C₁₋₄ alkyl esters of p-oxybenzoic acid, benzoic acid, alkali metal salts of benzoic acid and phenoxyethanol.

2. Use according to claim 1, wherein the alkyl galactoside represented by the formula (1) is at least one compound selected from the group consisting of , α,β-n-decyl galactoside, α,β-n-dodecyl galactoside, α,β-n-tetradecyl galactoside, α,β-n-hexadecyl galactoside, α,β-2-hexyldecyl galactoside and α,β-2-octyldodecyl galactoside.

3. Use according to any of the claims 1 or 2, wherein the preservative efficacy-enhancing composition comprises 0.01 to 30% by mass of an amphiphilic galactose derivative (A) and 0.01 to 1.0% by mass of an antiseptic agent (B).

4. Use according to claim 3, wherein the preservative efficacy-enhancing composition further comprises 0.01 to 50% by mass of a nonionic surfactant (C).

5. Use according to any of the claims 1 to 4, wherein the preservative efficacy-enhancing composition is contained in any of cosmetics, drugs, quasi-drugs, detergents and foods.

6. A method for enhancing a preservative efficacy of an antiseptic composition, comprising the step of allowing 0.01 to 30% by mass of an amphiphilic galactose derivative (A) to coexist with an antiseptic agent (B) in the composition
wherein the amphiphilic galactose derivative (A) is an alkyl galactoside represented by the following formula (1):
R-O- (G)ₙ (1)
wherein R is a linear or branched alkyl group having 6 to 36 carbon atoms which may be substituted; G is a galactose residue; and n is an integer of 1 to 20; and
wherein the antiseptic agent (B) is at least one compound selected from the group consisting of C₁₋₄ alkyl esters of p-oxybenzoic acid, benzoic acid, alkali metal salts of benzoic acid and phenoxyethanol.

## Patentansprüche

1. Verwendung eines amphiphilen Galactosederivats (A) als wirksamem Bestandteil zur Verbesserung der Konservierungseffizienz eines antiseptischen Mittels (B) in einer die Konservierungseffizienz steigernden Zusammensetzung,
wobei das amphiphile Galactosederivat (A) ein Alkylgalactosid der nachstehenden Formel (1) ist:
R-O-(G)ₙ (1)
worin R eine geradkettige oder verzweigte Alkylgruppe mit 6 bis 36 Kohlenstoffatomen, die substituiert sein kann, ist; G ein Galactoserest ist; und n eine ganze Zahl von 1 bis 20 ist; und
wobei das antiseptische Mittel (B) mindestens eine Verbindung, ausgewählt aus der Gruppe bestehend aus C₁₋₄-Alkylestern von p-Oxybenzoesäure, Benzoesäure,
Alkalimetallsalzen von Benzoesäure und Phenoxyethanol, ist.

2. Verwendung gemäss Anspruch 1, wobei das Alkylgalactosid der Formel (1) mindestens eine Verbindung, ausgewählt aus der Gruppe bestehend aus α,β-n-Decylgalactosid, α,β-n-Dodecylgalactosid, α,β-n-Tetradecylgalactosid, α,β-n-Hexadecylgalactosid, α,β-2-Hexyldecylgalactosid und α,β-2-octyldodecylgalactosid, ist.

3. Verwendung gemäss irgendeinem der Ansprüche 1 oder 2, wobei die die Konservierungseffizienz steigernde Zusammensetzung 0,01 bis 30 Masse-% eines amphiphilen Galactosederivats (A) und 0,01 bis 1,0 Masse-% eines antiseptischen Mittels (B) umfasst.

4. Verwendung gemäss Anspruch 3, wobei die die Konservierungseffizienz steigernde Zusammensetzung ferner 0,01 bis 50 Masse-% eines nicht-ionischen Tensids (C) umfasst.

5. Verwendung gemäss irgendeinem der Ansprüche 1 bis 4, wobei die die Konservierungseffizienz steigernde Zusammensetzung in Kosmetika, Arzneimitteln, Quasi-Arzneimitteln, Reinigungsmitteln und Lebensmitteln enthalten ist.

6. Verfahren zur Verbesserung der
Konservierungseffizienz einer antiseptischen Zusammensetzung, umfassend den Schritt, 0,01 bis 30 Masse-% eines amphiphilen Galactosederivats (A) mit einem antiseptischen Mittel (B) gemeinsam in der Zusammensetzung vorliegen zu lassen,
wobei das amphiphile Galactosederivat (A) ein Alkylgalactosid der nachstehenden Formel (1) ist:
R-O-(G)ₙ (1)
worin R eine geradkettige oder verzweigte Alkylgruppe mit 6 bis 36 Kohlenstoffatomen, die substituiert sein kann, ist; G ein Galactoserest ist; und n eine ganze Zahl von 1 bis 20 ist; und
wobei das antiseptische Mittel (B) mindestens eine Verbindung, ausgewählt aus der Gruppe bestehend aus C₁₋₄-Alkylestern von p-Oxybenzoesäure, Benzoesäure, Alkalimetallsalzen von Benzoesäure und Phenoxyethanol, ist.

## Revendications

1. Utilisation d'un dérivé de galactose amphiphile (A) en tant qu'ingrédient efficace pour améliorer l'efficacité de conservation d'un agent antiseptique (B) dans une composition améliorant l'efficacité de conservation,
dans laquelle le dérivé de galactose amphiphile (A) est un alkyl-galactoside représenté par la formule suivante (1) :
R-O-(G)n (1)
dans laquelle R est un groupe alkyle linéaire ou ramifié ayant 6 à 36 atomes de carbone qui peuvent être substitués ; G est un résidu de galactose ; et n est un nombre entier de 1 à 20 ; et
dans laquelle l'agent antiseptique (B) est au moins un composé choisi dans le groupe consistant en les alkylesters en C₁ à C₄ d'acide p-oxybenzoïque, l'acide benzoïque, les sels de métal alcalin d'acide benzoïque et le phénoxyéthanol.

2. Utilisation selon la revendication 1, dans laquelle l'alkyl-galactoside représenté par la formule (1) est au moins un composé choisi dans le groupe consistant en l'α,β-n-décyl-galactoside, l'α,β-n-dodécyl-galactoside, l'α,β-n-tétradécyl-galactoside, l'α,β-n-hexadécyl-galactoside, l'α,β-2-hexyldécyl-galactoside et l'α,β-2-octyldodécyl-galactoside.

3. Utilisation selon l'une quelconque des revendications 1 ou 2, dans laquelle la composition servant à améliorer l'efficacité de conservation comprend 0,01 à 30 % en poids d'un dérivé de galactose amphiphile (A) et 0,01 à 1,0 % en poids d'un agent antiseptique (B).

4. Utilisation selon la revendication 3, dans laquelle la composition pour améliorer l'efficacité de conservation comprend en outre 0,01 à 50 % en poids d'un tensioactif non ionique (C).

5. Utilisation selon l'une quelconque des revendications 1 à 4, dans laquelle la composition pour améliorer l'efficacité de conservation est contenue dans des produits cosmétiques, des médicaments, des quasi-médicaments, des détergents et des aliments.

6. Procédé pour améliorer l'efficacité de conservation d'une composition antiseptique, comprenant l'étape consistant à permettre à 0,01 à 30 % en poids d'un dérivé de galactose amphiphile (A) de coexister avec un agent antiseptique (B) dans la composition,
le dérivé de galactose amphiphile (A) étant un alkyl-galactoside représenté par la formule suivante (1) :
R-O-(G)n (1)
dans laquelle R est un groupe alkyle linéaire ou ramifié ayant 6 à 36 atomes de carbone qui peuvent être substitués ; G est un résidu de galactose ; et n est un nombre entier de 1 à 20 ; et
dans laquelle l'agent antiseptique (B) est au moins un composé choisi dans le groupe consistant en les alkylesters en C₁ à C₄ d'acide p-oxybenzoïque, l'acide benzoïque, les sels de métal alcalin d'acide benzoïque et le phénoxyéthanol
